(19) Europäisches Patentamt
European Patent Office
Office européen des brevets

(11) Publication number: 0 024 888
A2

(12) # EUROPEAN PATENT APPLICATION

(21) Application number: 80302895.0

(22) Date of filing: 21.08.80

(51) Int. Cl.[3]: C 07 C 131/00
C 07 D 521/00, A 01 N 35/10
A 01 N 43/00
//C07C49/76, (C07D521/00, 307/42)

(30) Priority: 24.08.79 US 69453
12.02.80 US 120806

(43) Date of publication of application:
11.03.81 Bulletin 81/10

(84) Designated Contracting States:
BE CH DE FR GB IT LI NL

(71) Applicant: MOBIL OIL CORPORATION
150 East 42nd Street
New York New York 10017(US)

(72) Inventor: Strong, Jerry Glen
2041 Castlebridge Road
Midlothian Virginia(US)

(72) Inventor: Paul, Jill Helaine
1375 River Road
Edgewater New Jersey(US)

(74) Representative: Cooper, John Anthony
Mobil Court 3 Clements Inn
London WC2A 2EB(GB)

(54) **Ketoximinoether insecticides.**

(57) There are now provided new specifically substituted methyl ethers of ketoximes, wherein one portion of the ketoxime is phenyl substituted by nitro, trifluoromethylthio, difluoromethylthio, 2,2-difluoromethylenedioxy, trifluoromethoxy, difluoromethoxy, haloalkoxy or sulfonamido, and the other is alkyl, which may contain substituents, such as halogen, hydroxy, alkoxy, alkylthio, alkylsulfonyl, nitro, carbalkoxy, acyl or cyano. There are also provided the method of combatting insects with these compounds and insecticidal compositions containing them and a suitable carrier.

EP 0 024 888 A2

Croydon Printing Company Ltd.

KETOXIMINOETHER INSECTICIDES

This invention is directed to certain ketoximinoethers having insecticidal activity.

The compounds of this invention have the general structure

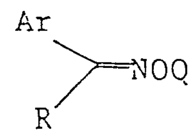

wherein Ar, R and Q are as defined hereinafter.

In U.S. Patent No. 3,980,799, there are disclosed, as synergists for insecticides and acaricides, compounds having the formula:

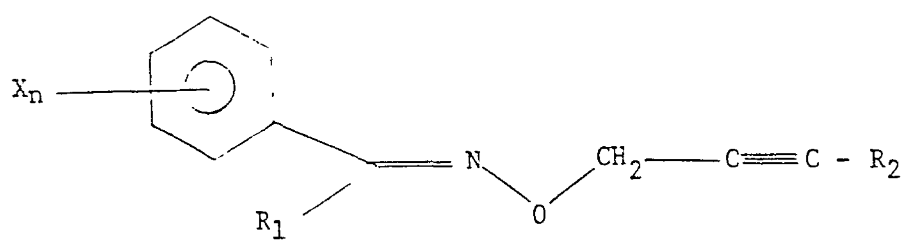

wherein $X_n$ is halogen, alkyl, methylenedioxy, etx., $R_1$ is $C_1$ to $C_4$ alkyl, and $R_2$ is hydrogen or halogen.

In British Patent No. 960,241, there are disclosed as synergists for insecticides, compounds having the formula:

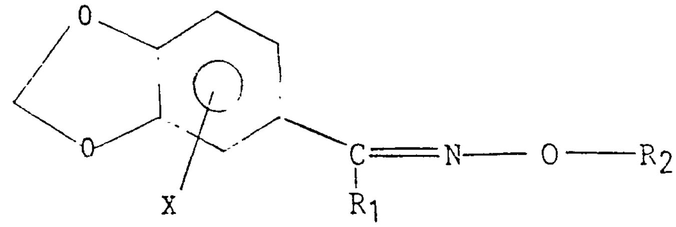

wherein X is hydrogen, n-propyl or alkyl, $R_1$ is hydrogen $C_1$ - $C_6$ alkyl or phenyl, and $R_2$ is alkyl, alkenyl, aralkyl or cycloalkyl.

In U.S. Patent No. 4,079,149 it is disclosed that useful insecticidal properties are possessed by benzyl oxime ethers of the formula

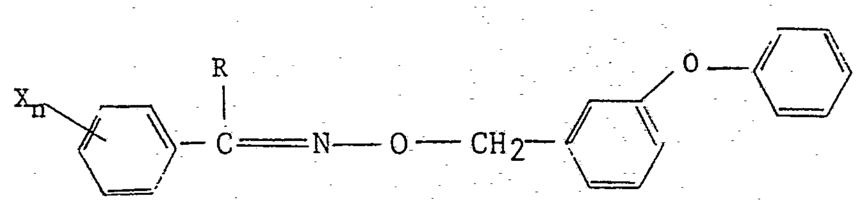

wherein n is 0, 1 or 2, X is halogen, or is alkyl, or alkoxy of from one to three carbon atoms, and R is alkyl or alkenyl of from two to seven carbon atoms, optionally substituted by from one to a plurality of halogen atoms, or is cyclopropyl, optionally substituted by from one to four methyl groups and one or two halogen atoms.

This invention provides compounds having the formula:

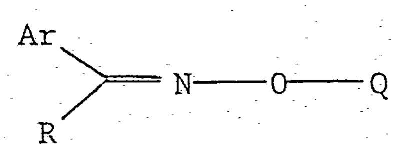

wherein Ar is phenyl substituted in the 2- to 6-position with nitro, trifluoromethylthio, difluoromethylthio, 2,2-difluoromethylenedioxy, trifluoromethoxy, difluoromethoxy, haloalkoxy or sulfonamido; R is $C_2$ - $C_6$ alkyl, $C_3$ - $C_6$ cycloalkyl or $C_2$ - $C_6$ alkenyl any of which can be substituted by halogen, hydroxy, alkoxy, alkylthio, alkylsulfonyl, cyano, nitro,

carboalkoxy or acyl and Q is:

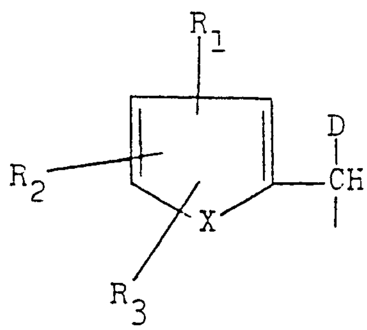

or

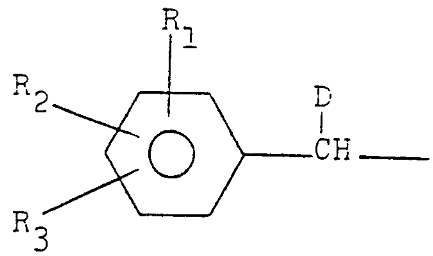

wherein X is oxygen or sulfur; D is hydrogen, cyano, thiocarboxamide alkyl or ethynyl; $R_1$ and $R_2$ are the same or different and are hydrogen, halogen, alkyl, alkenyl, alkynyl, methylenedioxy, haloalkyl, haloalkenyl or cyano; and $R_3$ is hydrogen or -ZY wherein Z is oxygen, sulfur or methylene and Y is hydrogen, alkyl, alkenyl, alkynyl or phenyl on which can be substituted alkyl, alkenyl, alkoxy, chlorine, bromine or fluorine; or

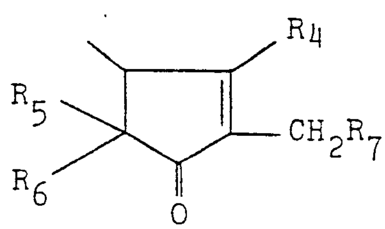

wherein $R_4$ is hydrogen or methyl; $R_5$ and $R_6$ are the same or different and are hydrogen or alkyl and $R_7$ is ethenyl, ethynyl or butadienyl; or

$$R_8CH_2-$$

wherein $R_8$ is phthalimido, di- or tetra-hydrophthalimido or cyano; or

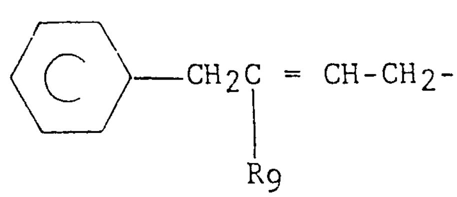

wherein $R_9$ is hydrogen, halogen or methyl; or

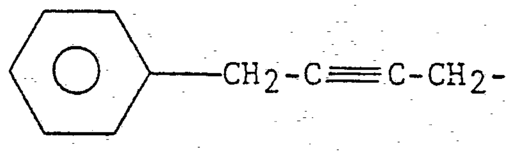

Preferably, when R is alkyl, alkenyl or cycloalkyl, Ar cannot be phenyl or phenyl substituted by halogen, alkyl or alkoxy. More preferably, Ar is phenyl substituted in the 2- to 6- position with 1-5 amino, alkylamino, dialkylamino, alkylthio, methylene dioxy, ethylene dioxy, isopropylene dioxy, trifluoromethyl, haloalkyl, alkylthioalkyl, alkenyl, alkynyl, cyano, cyanoalkyl, carbo alkoxy, alkylsulfonyl, alkoxyalkyl, haloalkenyl or acyl; indanyl; naphthyl; benzofuryl; benzodihydrofuryl; benzothienyl; heterocyclic aryl or heterocyclic aryl substituted with halogen, alkyl, nitro, amino, alkylamino, dialkylamino, alkoxy, methylenedioxy, alkylthio, haloalkyl, alkylthioalkyl, lakenyl, alkynyl, cyano, cyanoalkyl, carboalkoxy, alkylsulfonyl, alkoxy alkyl, haloalkenyl or acyl.

Typical examples of the Q substituent are:

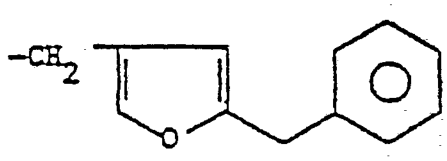

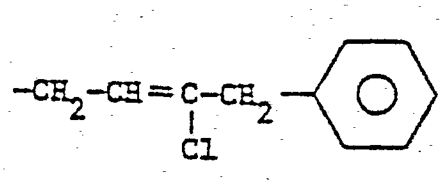

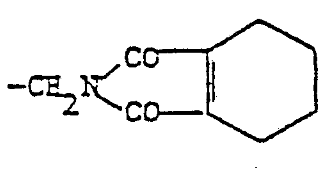

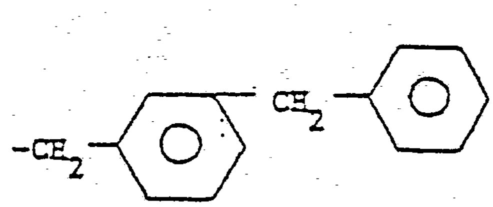

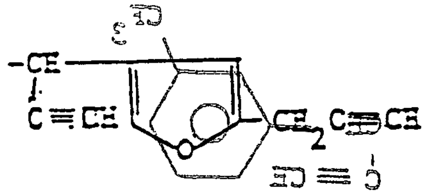

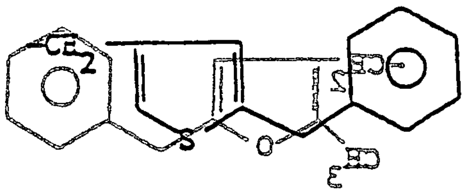

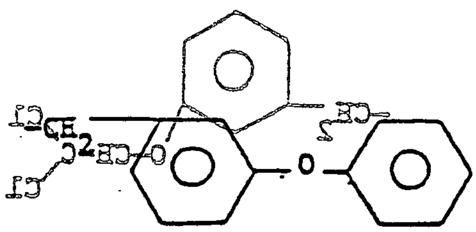

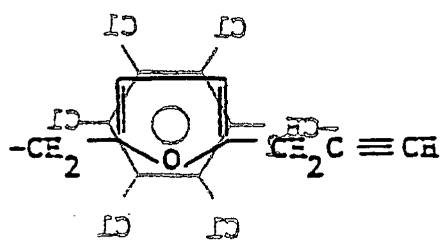

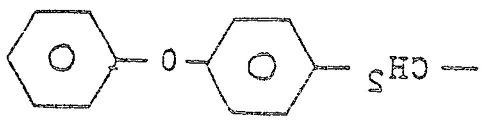

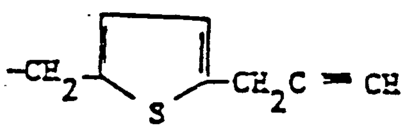

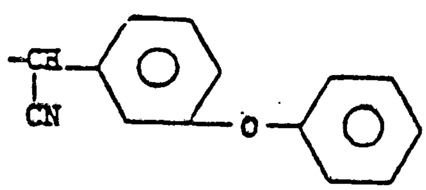

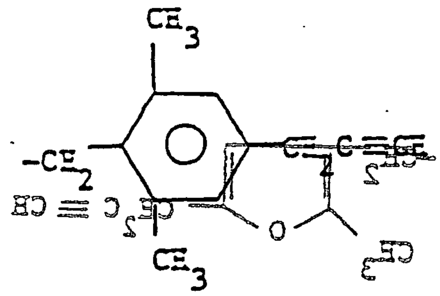

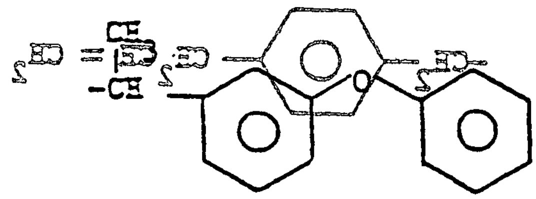

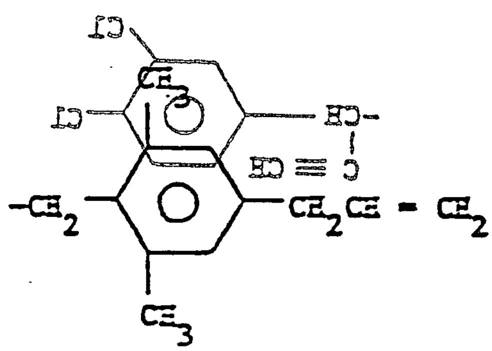

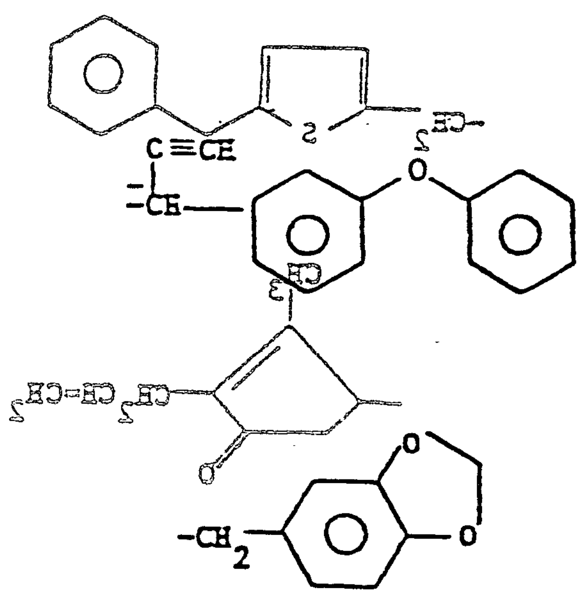

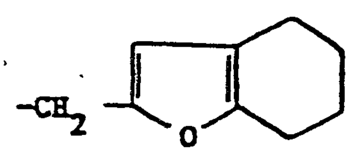

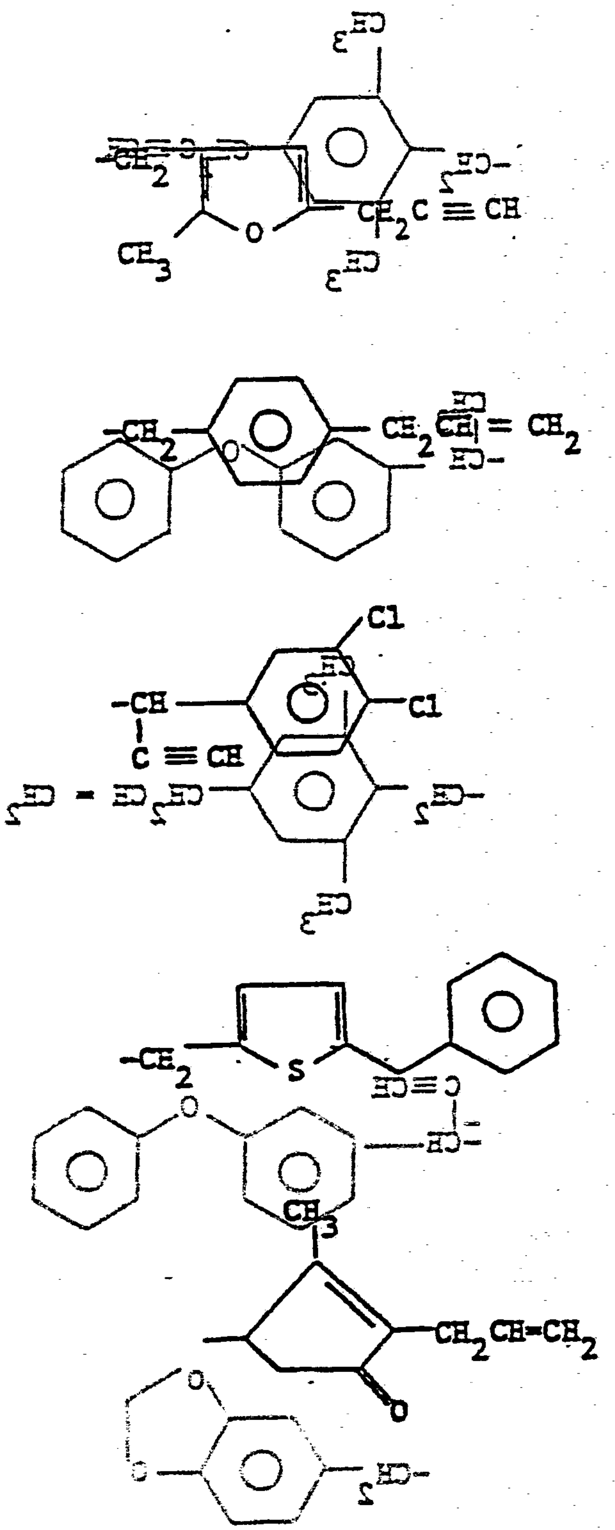

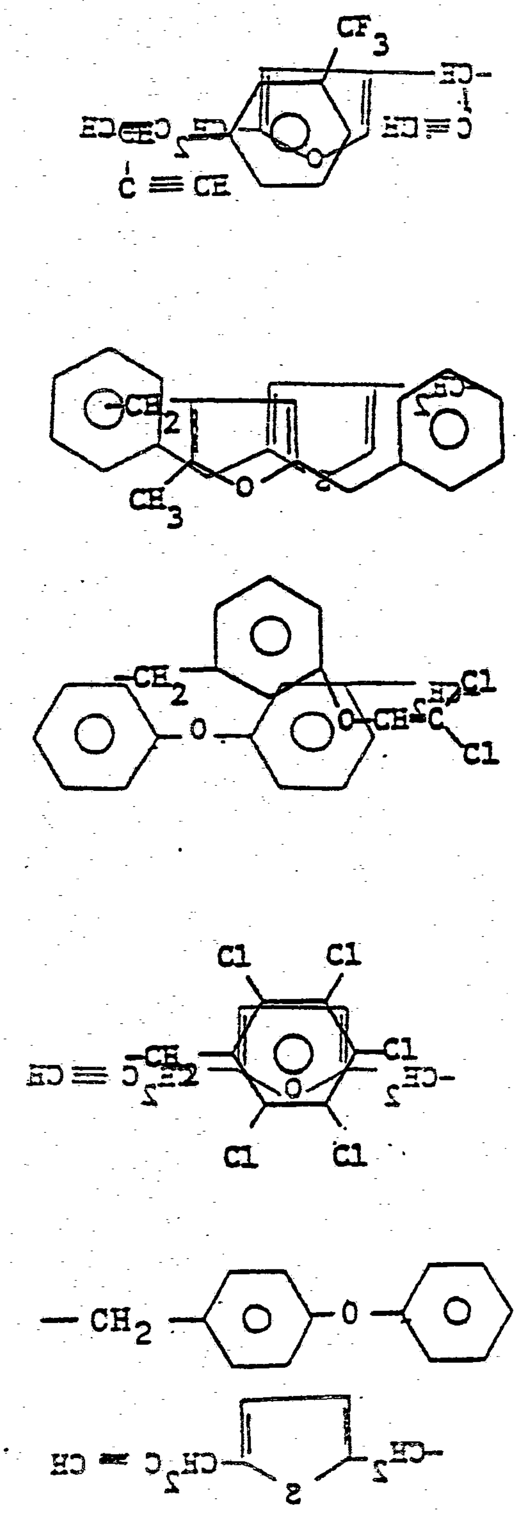

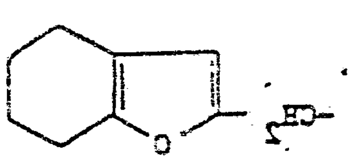

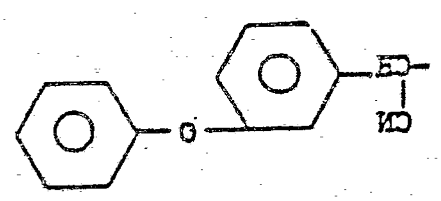

Examples of compounds of this invention are compounds having the structural formulae:

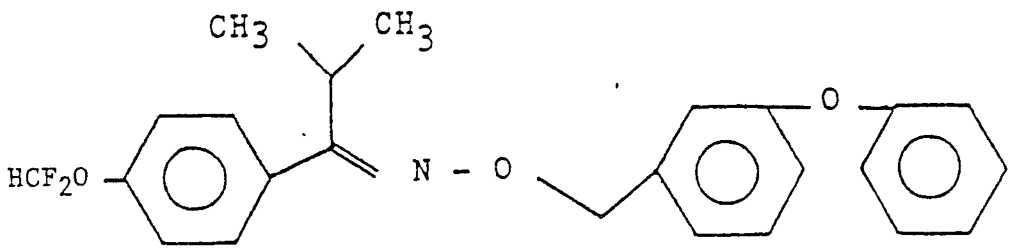

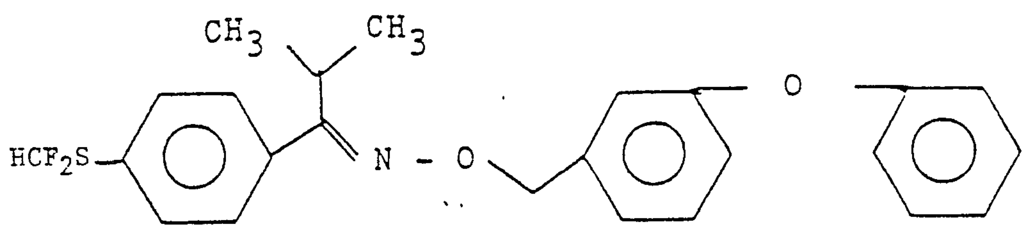

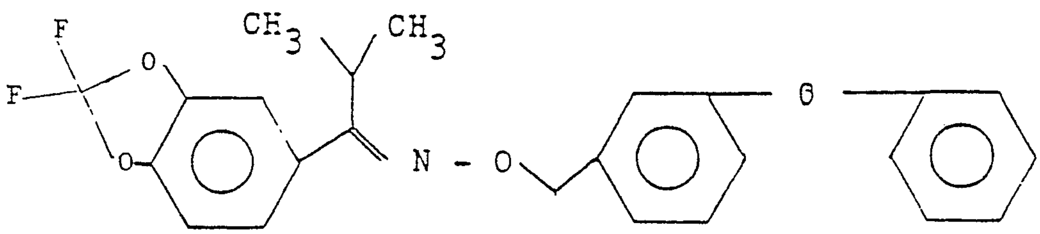

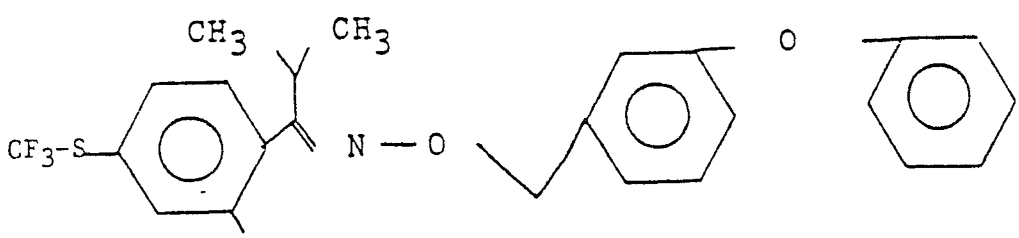

## SYNTHESIS METHODS

In general the compounds of this invention ai prepared by two methods:

(a) Condensation of an aryl alkyl ketoxime with an alkyl halide in the presence of a base and a suitable solvent (general procedure E).

(b) Condensation of an isolated alkali metal ketoximate salt with an alkyl halide in an appropriate solvent (general procedure F).

The ketoximes are prepared from aryl alkyl ketones and hydroxylamine hydrochloride in a manner familiar to those skilled in the art and according to general procedure D.

The aryl alkyl ketones are obtained from commercial sources or are synthesized from available aromatics and carboxylic acid chlorides or from a benzonitrile and a Grignard reagent, as described in general procedures A, B and C.

The alkyl halides are obtained from commercial sources or are synthesized from available alcohol and a halogenating agent (e.g. thionyl chloride, phosphorous tribromide, etc.) in a manner familiar to those skilled in the art.

### General Procedure A for Aryl Alkyl Ketones

A solution of 3.0 mole of the substituted benzene and 0.8 mole of alkyl acid chloride is cooled to -5°C. Aluminum chloride (0.9 mole) is added portionwise, with vigorous stirring, while maintaining the reaction temperature at 0°C. The reaction mixture is

brought to room temperature and allowed to stir overnight, whereupon the whole is poured into 450 ml of concentrated HCl and 1200 ml of ice-$H_2O$. The aqueous mixture is extracted two times with 350 ml portions of $CHCl_3$. The combined organic extracts are washed with 5% NaOH, $H_2O$, dried over $MgSO_4$ and concentrated under reduced pressure. The residue is distilled via short path to obtain 0.64 - 0.68 mole of pure product.

General Procedure B for Aryl Alkyl Ketones

Typical Example

Preparation of 5-bromo-2-isobutyrothiophenone

A solution of 8.2 g (50 mole) of 2-bromothiophene and 5.3 g (50 mole) of isobutyryl chloride in 40 ml of $CS_2$ is cooled to 0°C. Aluminum chloride 7.3g (55 mole) is added portionwise with vigorous stirring, while maintaining the reaction temperature at 0°C. The remaining manipulations are carried out as described in Procedure A to yield 6.2 g of pure product. Bp 70°C (0.02 mm); IR (neat) ($cm1^-$); 1666 (s), 1527 (s), 1412 (s), 1222 (s), 980 (s) nmr ($CDCl_3$) (ppm) 1.17 (6H, d), 3.33 (1H, m), 7.13 - 7.60 (2H, m).

General Procedure C for Aryl Alkyl Ketones

Typical Example

Preparation of m-Methyl Isobutyrophenone

A solution of 23.4 g (0.2 mole) of m-methylbenzonitrile in 50 ml of dry tetrahydrofuran is cooled to 10°C. A 2.2M solution (90 ml, 0.2 mole) of isopropyl magnesium chloride in ether is added dropwise, while maintaining the reaction temperature below 30°C. The ether is removed (by distillation) and the reaction mixture

heated to 70°C for 4 hours. Upon cooling to room temperature, 100 ml of aqueous 6 N HCl is added dropwise. Reaction mixture is refluxed for 2 hours. The resulting two layers are separated and the aqueous layer is extracted three times with 200 ml portions of ether. The combined extracts are washed once with $H_2O$, dried over $MgSO_4$ and concentrated under reduced pressure. The residue is distilled using a vigreux column to obtain 23.6 g (73% yield) of pure product.

## General Procedure D for Ketone Oximes

In general, the aryl alkyl ketones prepared by general procedures A, B and C, were dissolved in ethanol and refluxed 3 - 5 hours in the presence of an excess of hydroxylamine hydrochloride and potassium hydroxide. The reaction mixture is concentrated and the oxime or its potassium hydroxide. The reaction mixture is concentrated and the oxime or its potassium salt is extracted into an organic solvent or $H_2O$, followed by acidification with concentrated HCl, respectively. Recrystallization from either hexane, hexane/$CHCl_2$ or $EtOH/H_2O$ followed.

General Procedure E for Oximinoethers

In general, 0.02 mole of the ketone oximes prepared by general procedure D, were dissolved in 10 ml of toluene and added dropwise to a slurry of 0.02 mole of NaH (57% in mineral oil) in 10 ml toluene and 4 ml of DMF. The mixture is warmed to 50°C until $H_2$ evolution ceases, whereupon 0.02 mole of alkyl halide is added dropwise at 30°C. The reaction mixture is stirred overnight at room temperature and quenched by addition of 50 ml of $H_2O$ and 100 ml of toluene. The organic layer is washed with 5%, 50:50 aqueous/ethanol NaOH, followed by $H_2O$, dried over $MgSO_4$, and concentrated under reduced pressure to 0.015 - 0.017 mole of crude product. Chromatography on silica gel, using 50:50 $CH_2Cl_2$/hexane as eluent provides a highly purified product.

General Procedure F for Oximinoethers

A solution of 0.02 mole of ketoxime in 20 ml of ethanol is added to a freshly prepared ethanolic solution of 0.02 mole NaOEt. The mixture is stirred at room temperature for 30 minutes and concentrated under reduced pressure to dryness. The resulting sodium oximate salt is dissolved in a minimum volume of 90% DMF and 10% t-butanol, whereupon 0.02 mole of alkyl halide (neat) is added dropwise, causing an exotherm of 8°C. The reaction mixture is stirred overnight at room temperature and poured into $H_2O$. The resulting oil is extracted two times into toluene. The combined organic layers are washed with 5% NaOH (50:50 $H_2O$/ethanol) $H_2O$, dried over $MgSO_4$, and concentrated under reduced pressure to yield 0.15 - 0.19 mole of >90% pure product.

The oximino function of the compounds of this invention provides for the possibility of two geometrical isomers. This is shown in the following isomeric structures of a typical compound of this invention:

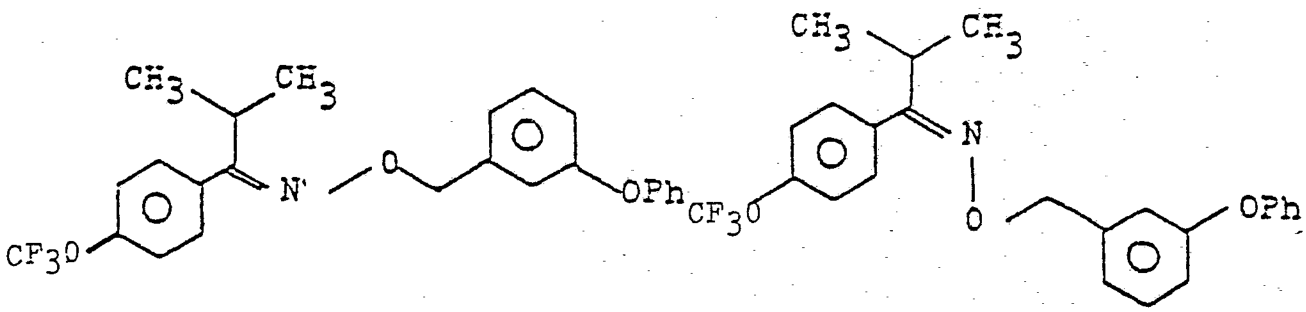

The O- (m-phenoxy) benzyl may be either cis to the isopropyl group (E or "syn" isomer) or cis to the p-trifluoromethoxy phenyl ring (Z or "anti" isomer). Many compounds of this type occur as mixtures of isomers. In general, E isomer compounds or mixtures rich in E isomer compounds show the greater activity against some insect species and may be preferable. Z isomer compounds or mixtures rich in Z isomer compounds, however, do have substantial insecticidal activity. Accordingly, compounds having both isomeric structures of this invention are contemplated, regardless of isomer type or of isomer content in isomer mixtures.

The following compounds were prepared:

Example 1

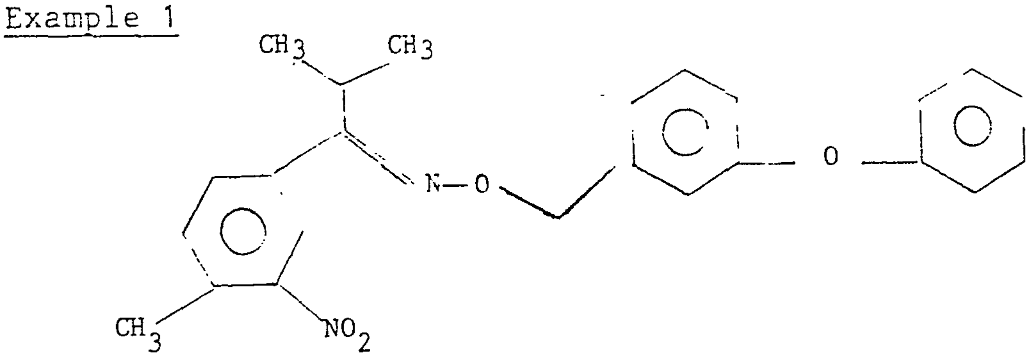

m-Methyl isobutyrophenone was nitrated with a mixture of nitric and sulfuric acids at a temperature of about 0°C. The product was separated from the reaction mixture by pouring the mixture into iced water and cooling in an ice/salt bath whereupon the oily product solidified and could be filtered off. The nitrated product, 3-nitro-4-methyl-isobutyrophenone, was essentially pure.

The ketoxime was produced from the ketone by General Procedure D above above and the oxime converted to the ketoximinoether by General Procedure E above, with the product having an E/Z ratio of 50:50; IR (lamda, max) $cm^{-1}$: 1585(s), 1525(s), 1492(s), 1345.

Example 2

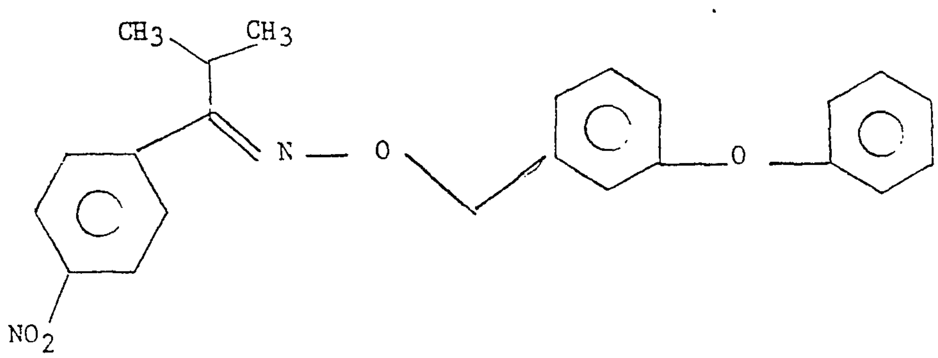

m-Nitro isobutyrophenone was prepared by reaction between p-nitrobenzoyl chloride and the appropriate enamine (N-isobutenyl morpholine).

p-Nitrobenzoyl chloride (3.69g.) was added dropwise to a solution of the enamine (2.8g.) in tetrahydrofuran at 5°C. After refluxing for 2 hours the mixture was cooled to room temperature and diethyl ether added. The insoluble salts were filtered off and then dissolved in water and diethyl ether addded. After stirring for 2 hours the organic layer was separated and the aqueous layer washed several times with diethyl ether after which the ether extracts were dried and concentrated. The crude reaction material (2.6g.) was then treated with 5 ml. of concentrated HCl and 10 ml. water and stirred overnight at room temperature after which the mixture was diluted with ether and poured into water. The ether layer was separated and combined with an ether extract of the aqueous layer. After washing and drying, the ether was removed to yield the product, m-nitro-isobutyrophenone (1.8g.), m.p. 49°C.

The ketoxime was then prepared from the ketone by General Procedure D above and the ketoximinoether by General Procedure E above, the product having an E/Z ratio of 50:50. IR (lamda, max) $cm^{-1}$: 1585(s), 1520(s), 1490(s), 1345(s).

<u>Example 3</u>

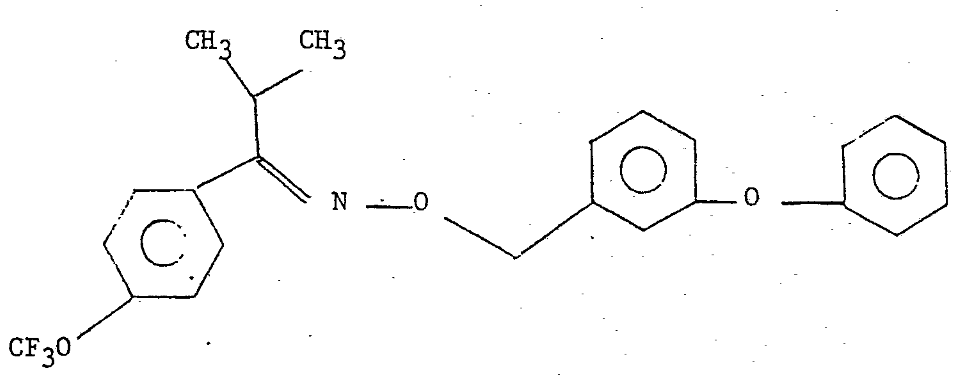

p-Trifluoromethoxy benzonitrile was prepared by the reaction of triphenyl phosphine with p-trifluoromethoxy benzamide. This preparation was taken from Eisaku Yamato and Shigehiko Sugasawa: Tetrahedron Letters No. 50, pp. 4383-4384, 1970. "Preparation of Nitrile from Primary Amide". It was chosen because it is a mild dehydration method which should not cause cleavage of the $-OCF_3$ group.

Triphenylphosphine (13.8g) was dissolved partially in 40 ml. $CCl_4$. To this was added dropwise p-trifluoromethoxy benzamide (5.0g) which was dissolved in 40 ml. dry THF. The literature noted a slight exotherm, which was not observed. The entire mixture was then warmed to $50^o$-$60^o$ for about 2 hours. The solution was at first turbid, then completely colorless. After approximately 1/2 hr., a fine white precipitate, thought to be the triphenylphospheneoxide, was observed. The product was filtered and the filtrate was distilled.

Using General Procedure C above, the p-trifluoromethoxy benzonitrile was reacted with isopropyl magnesium chloride to produce the corresponding ketone. The ketoxime was then prepared from the ketone by General Procedure D and the ketoxime converted to the ketoximinoether by General Procedure E. IR (lamda, max), $cm^{-1}$: 1585(s), 1492(s), 1215, 1250 (broad, s).

Example 4

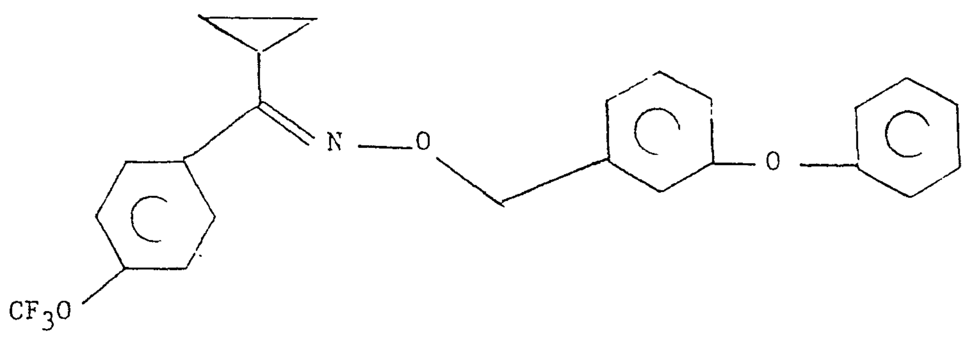

p-Trifluoromethoxybenzonitrile was prepared in the manner described in Example 3 above and reacted with cyclopropyl magnesium bromide accordingly to General Procedure C to produce the ketone which was then converted to the ketoximinoether by General Procedures D and E, giving a product with an E/Z ratio of 70/30, IR (lamda, max), $cm^{-1}$: 1585(s), 1492(s), 1215, 1250 (broad, s).

Example 5

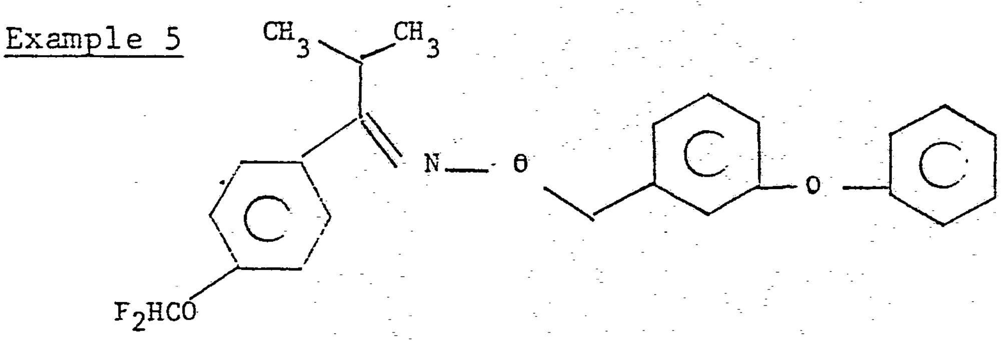

p-Hydroxy isobutyrophenone was prepared by the reaction of phenol (9.4g.) with isobutyric acid (17.6g) in the presence of boron trifluoride which was bubbled through a mixture of the phenol and the acid in a 3-necked flask fitted with gas dispersion tube, stirrer and thermometer. The mixture was maintained at a temperature of less than 5°C. and after adding the boron trifluoride over a period of 1.5 hours the mixture was warmed to room temperature and then heated to 70° for 2 hours after which it was allowed to cool. After chilling to 0°C, water (125 ml.) was added dropwise and the oily product extracted twice into diethyl ether. The ether extracts were washed three times with 5% sodium bicarbonate and the oily product separated.

p-Difluoromethoxy isobutyrophenone was prepared from the oil product by reaction with difluorochloromethane in the presence of sodium

hydroxide. The oily product (5.0g.) dissolved in 25 ml. dioxan was placed in a 3-neck flask fitted with gas inlet, gas outlet, magnetic stirrer and therometer together with sodium hydroxide (6.0g.) dissolved in water (20 ml.) the mixture was heated to 70°C whereupon a stream of chlorodifluoromethane was bubbled through the mixture, causing a 5°C exotherm. The gaseous halocarbon was added for 30 minutes after which the reaction mixture was cooled to room temperature and the upper layer separated and combined with ether extracts of the basic layer. The organic layers were washed with brine and water, dried and evaporated to give a dark brown liqud product (4.3g.) The crude product was distilled on kieselguhr at 80-90°C at 53.3 Pa to yield the pure product (3.5g.)

The oxime was prepared from the ketone by General Procedure D and the oxime ether from the oxime by General Prodcedure E. IR (lamda, max), $cm^{-1}$: 1585(s), 1492(s), 1215, 1250 (broad, s), 100% E isomer.

The compounds of this invention have been found to exhibit considerable biological activity. They are especially potent pesticides when used to control or combat important agricultural pests. These compounds can be used in various ways to achieve biological action. They can be applied per se, as solids or in varporized form, but are preferably applied as the toxic components in pesticidal compositions of the compound and a carrier. The compositions can be applied as dusts, as liquid sprays or as gas-propelled sprays and can contain, in addition to a carrier, additives such as emulsifying agents, wetting agents, binding agents, gases compressed to the liquid state, odorants, stabilizers and the like. A wide variety of liquid and solid carriers can be used in the pesticidal compositions.

Non-limiting examples of liquid carriers include water; organic solvents such as alcohols, ketones, amides and esters; mineral oils such as kerosene, light oils and medium oils, and vegetable oils such as cottonseed oil. Non-limiting examples of solid carriers include talc, bentonite, diatomaceous earth, pyrophylite, fullers earth, gypsum, flours derived from cotton seeds and nut shells and various natural and synthetic clays having a pH not exceeding about 9.5.

The amount of the compounds of this invention utilized in pesticidal compositions will vary rather widely. It depends to some extent upon the type of composition in which the material is being used, the nature of the condition to be controlled and the method of application (i.e., spraying, dusting etc.). In the ultimate pesticidal composition, as applied in the field, pesticide concentrations as low as 0.0001 weight percent of the total composition can be used. In general, compositions, as applied, containing about 0.05 weight percent pesticide in either liquid or solid carrier give excellent results. In some cases, however, stronger dosages up to about 10 weight percent may be required.

In practice, pesticidal compositions are usually prepared in the form of concentrates, which are diluted in the field to the concentration desired for application. For example, the concentrate can be a wettable powder containing large amounts of the compound of this invention, a carrier (e.g., attapulgite or other clay), and wetting and dispersing agents. Such powders can be diluted prior to application, by dispersing it in water to obtain sprayable suspension containing the concentration of pesticide desired for application. Other concentrates can be solutions that can be later diluted,

e.g., with kerosene. Thus, it is within the contemplation of this invention to provide pesticidal compositions containing up to about 80 percent, by weight of the composition, of a pesticidal compound of this invention. Accordingly, depending upon whether it is ready for application or it is in oncentrated form, the contemplated pesticidal compositions contain between about 0.0001 percent and about 80 percent, by weight of the compositions, of a pesticidal compound of this invention and a carrier, liquid or solid, as defined hereinbefore.

## INSECTICIDE TEST METHODS

### Bait Test [Housefly (adult)]

#### Method of Treatment

One milliliter of an aqueous solution of suspension of the candidate compound is pipetted into a 9 cm petri dish containing filter paper and 0.1 gm granular sugar. Ten adults are admitted and the dish is closed.

#### Method of Recording Results

Mortality is recorded after 24-75 hours. Compounds which produce 90% mortality are reevaluated at lower concentrations in secondary tests. Mode of action may be by stomach poison, contact or vapor.

### Larvicide/Growth Regulant Test (Yellow Fever Mosquito larvae)

Method of Treatment

The rearing medium is treated prior to infestation. For mosquito larvae this consists of 10 ml of water containing 10 ppm of the candidate compound in a plastic cup. Food is added after infesting with 5 last instar larvae. The cup is then capped.

Method of Recording Results

Mortality (larvicide) is recorded after 24 hours.

Stomach Poison - Foliar Dip Test

Primary Screen

Southern Armyworm, (Larva)
Mexican Bean Beetle (Larva)

Method of Treatment

Lima bean leaves of a uniform size are momentarily dipped in a 500 ppm water-acetone of the test material. Treated leaves are placed on moistened filter paper in 9 cm petri dishes and allowed to air dry and then are infested. The dishes are then closed.

Method of Recording Results

Mortality is recorded 72 hours after infestation. Compounds active at 500 ppm are retested at 100 and 10 ppm.

All test results are recorded as percent mortality. In the tabulation of data, the insect species are abbreviated as follows: Housefly (HF), Mexican Bean Beetle (MB), Southern Armyworm (SA) and Yellow Fever Mosquito (YF).

The compounds of Examples 1 through 5 were subjected to the aforedescribed insecticide test. Test concentrations and results are set forth in the Table below:

TABLE

| COMPOUND | RATE (PPM) | HF | MB | SA |
|---|---|---|---|---|
| Example 1 | 500 | 20 | 100 | 70 |
| | 100 | 5 | 100 | 15 |
| | 10 | 0 | 55 | - |
| Example 2 | 500 | 100 | 100 | 100 |
| | 100 | 85 | 100 | 80 |
| | 10 | 35 | 55 | 5 |
| Example 3 | 500 | 100 | 100 | 100 |
| | 100 | 100 | 100 | 100 |
| | 10 | 50 | 100 | 95 |
| Example 4 | 500 | 100 | 100 | 100 |
| | 100 | 100 | 100 | 100 |
| | 10 | 100 | 100 | 100 |
| Example 5 | 100 | 100 | 100 | 100 |
| | 10 | 95 | 85 | 0 |
| | 5 | 65 | 75 | - |

CLAIMS

1. Compounds having the formula:

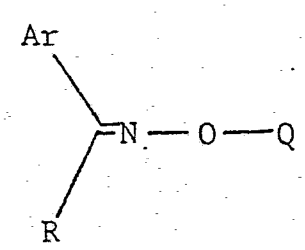

wherein Ar is phenyl substituted in the 2- to 6-position with nitro, trifluoromethylthio, difluoromethylthio, 2,2-difluoromethylenedioxy, trifluoromethoxy, difluoromethyoxy, haloalkoxy or sulfonamido; R is $C_2$ - $C_6$ alkyl, $C_3$ - $C_6$ cycloalkyl or $C_2$ - $C_6$ alkenyl any of which can be substituted by halogen, hydroxy, alkoxy, alkylthio, alkylsulfonyl, cyano, nitro, carboalkoxy or acyl; and Q is:

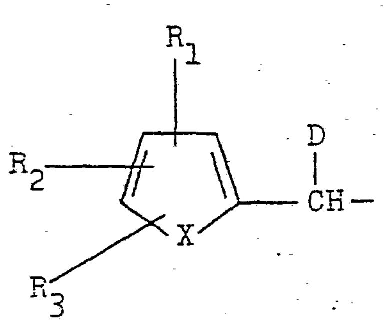

or

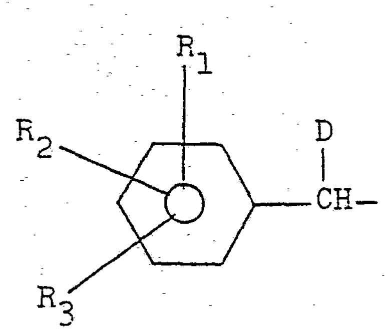

wherein X is oxygen or sulfur; D is hydrogen, cyano, thiocarboxamido, alkyl or ethynyl, $R_1$ and $R_2$ are the same or different and are hydrogen, halogen, alkyl, alkenyl, alkynyl, methylenedioxy, haloalkyl, haloalkenyl or cyano; and $R_3$ is hydrogen or -ZY wherein Z is oxygen, sulfur or methylene and Y is hydrogen, alkyl, alkenyl, alkynyl or phenyl on which can be substituted alkyl, alkenyl, alkoxy, chlorine, bromine or fluorine; or

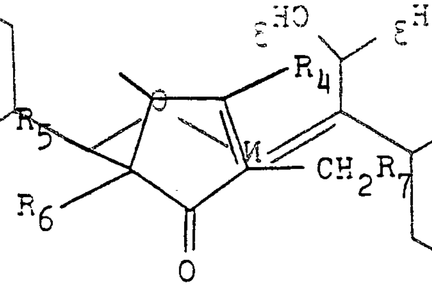

wherein $R_4$ is hydrogen or methyl; $R_5$ and $R_6$ are the same or different and are hydrogen or alkyl; and $R_7$ is ethenyl, ethynyl or butadienyl; or

$R_8CH_2-$

wherein $R_8$ is phthalimido, di- or tetra-hydrophthalimido or cyano; or

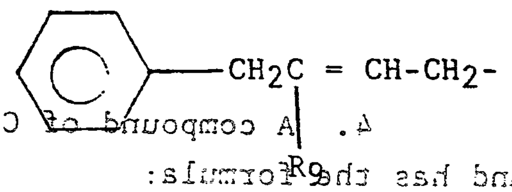

wherein $R_9$ is hydrogen, halogen or methyl; or

$-CH_2-C \equiv C-CH_2-$

2. A compound of Claim 1 wherein said compound has the formula:

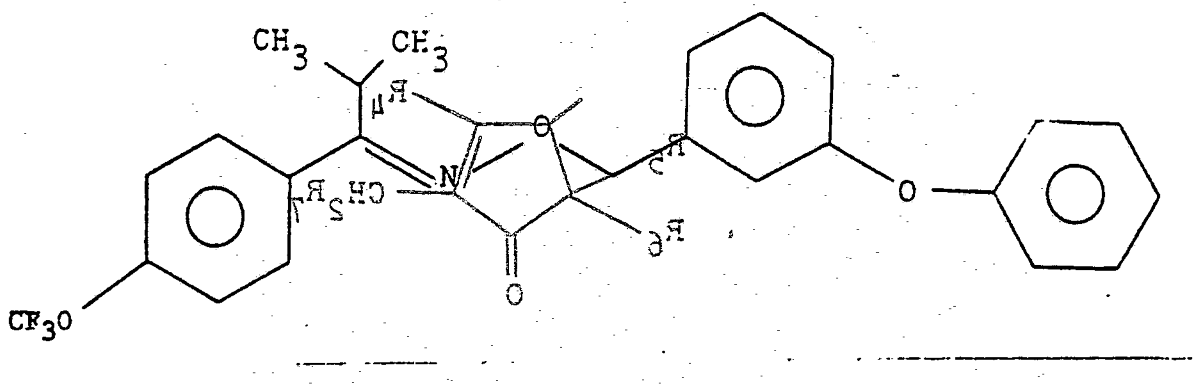

3. A compound of Claim 1 wherein said compound has the formula:

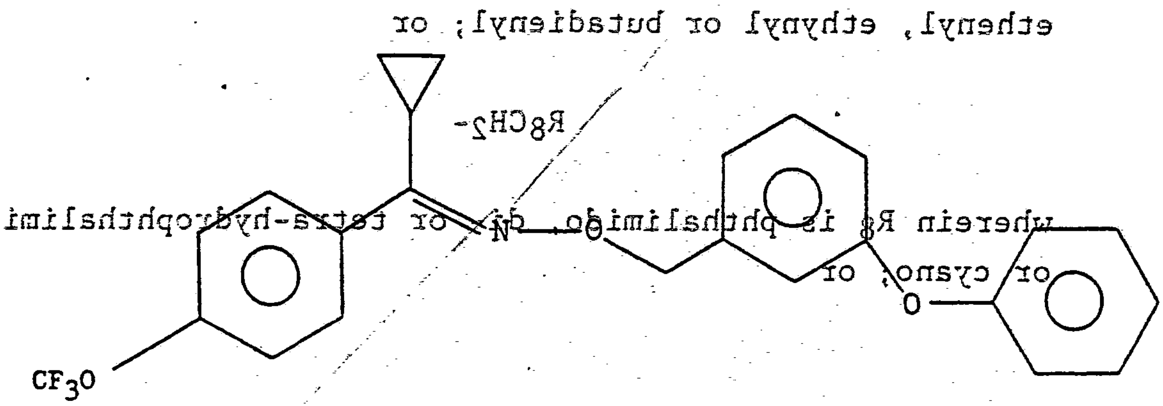

4. A compound of Claim 1 wherein said compound has the formula:

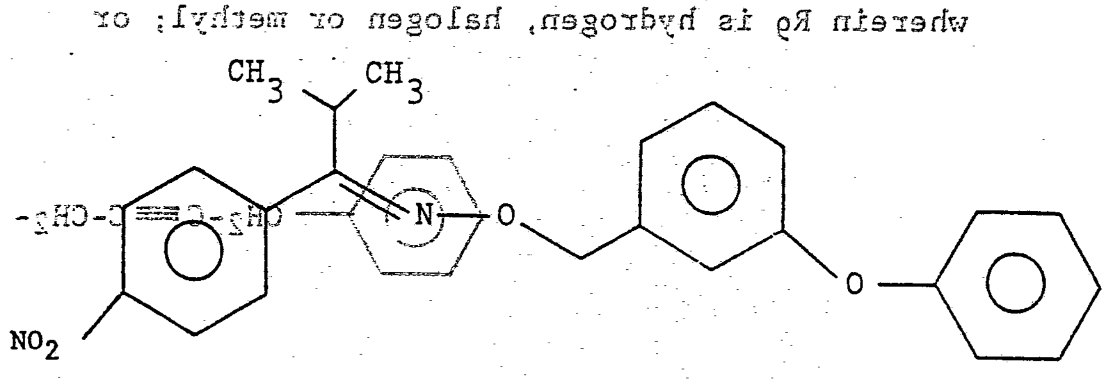

5. A compound of Claim 1 wherein said compound has the formula:

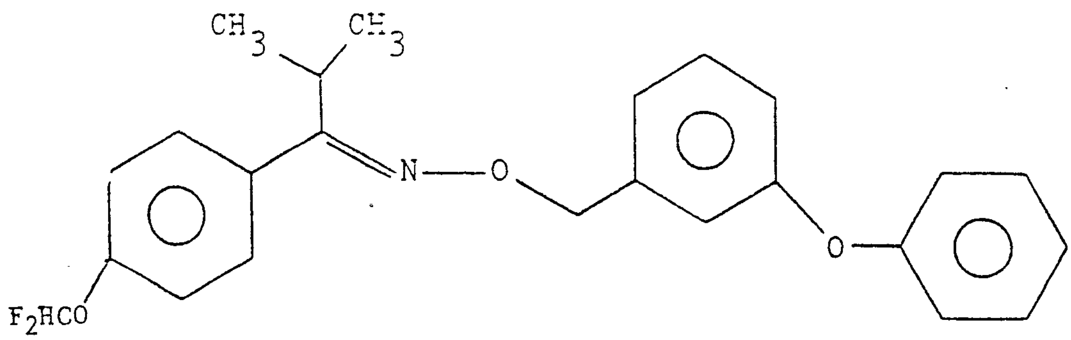

6. A compound of Claim 1 wherein said compound has the formula:

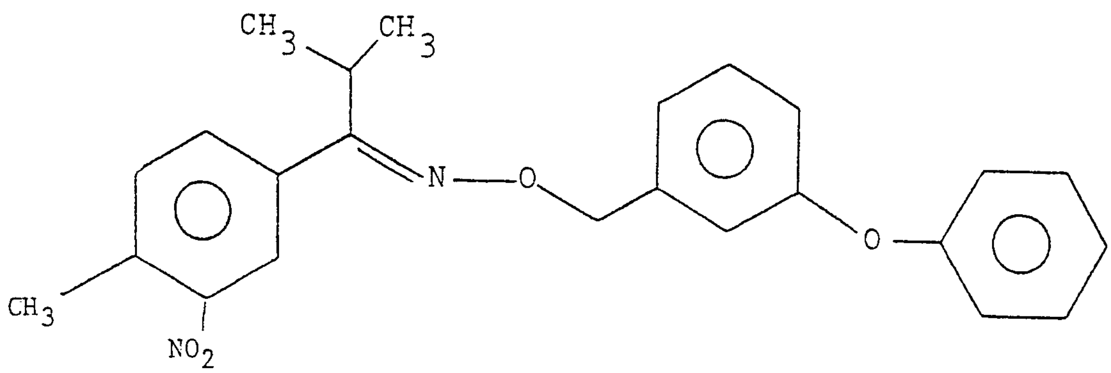

7. An insecticidal composition comprising a carrier and an insecticidally effective amount of a compound of Claim 1.

8. An insecticidal composition comprising a carrier and an insecticidally effective amount of a compound of Claim 2.

9. An insecticidal composition comprising a carrier and an insecticidally effective amount of a compound of Claim 3.

10. An insecticidal composition comprising a carrier and an insecticidally effective amount of a compound of Claim 4.

11. An insecticidal composition comprising a carrier and an insecticidally effective amount of a compound of Claim 5.

12. An insecticidal composition comprising a carrier and an insecticidally effective amount of a compound of Claim 6.

13. The method for combatting insects which comprises contacting them with an insecticidally effective amount of a compound of Claim 1.

14. The method for combatting insects which comprises contacting them with an insecticidally effective amount of a compound of Claim 2.

15. The method for combatting insects which comprises contacting them with an insecticidally effective amount of a compound of Claim 3.

16. The method for combatting insects which comprises contacting them with an insecticidally effective amount of a compound of Claim 4.

17. The method for combatting insects which comprises contacting them with an insecticidallly effective amount of a compound of Claim 5.

18. The method for combatting insects which comprises contacting them with an insecticidally effective amount of a compound of Claim 6.